# EUROPEAN PATENT APPLICATION

(11) **EP 2 143 439 A1**
(43) Date of publication of application: **13.01.2010**
(21) Application number: 08721551.3
(22) Date of filing: 07.03.2008
(51) Int. Cl.: A61K 38/22, A61P 37/06, A61P 43/00

(54) **PROTECTIVE AGENT FOR TRANSPLANTED ORGAN**

(30) Priority: 09.03.2007 JP 2007059632
(71) Applicant: National University Corporation Obihiro University of Agriculture and Veterinary Medicine, Obihiro-shi, Hokkaido 080-8555 (JP); Chugai Seiyaku Kabushiki Kaisha, Kita-ku Tokyo 115-8543 (JP)
(72) Inventor: SUZUKI, Hiroshi, Obihiro-shi Hokkaido 080-0028 (JP); SAITO, Hideki, Tokyo 103-8324 (JP)
(74) Representative: Spilgies, Jan-Hendrik
(86) International application number: PCT/JP2008/054133
(87) International publication number: WO 2008/111503

(57) **Abstract**

Compositions for protecting transplanted organs, promoting survival of transplanted organs or preserving organs for transplant containing erythropoietin (EPO) as an active ingredient are provided.

## Description

### TECHNICAL FIELD

The present invention relates to novel compositions for protecting transplanted organs, especially compositions having a survival-promoting effect on transplanted organs.

### BACKGROUND ART

Erythropoietin (hereinafter sometimes referred to as EPO) is an acidic glycoprotein hormone which promotes differentiation and proliferation of erythroid progenitor cells, and mainly produced by the kidney. EPO plays a central role in maintaining erythrocyte homeostasis in vivo, and it is clinically used for the treatment of anemia and pre- and postoperative management.

It has been known that EPO functions not only as a hematopoietic factor but also to inhibit apoptosis or protect tissues in nerve cells, myocardial cells, renal proximal tubular epithelial cells, etc. (PANS 100: 4802-4806, 2003, etc.). Such two functions of EPO are attributed to two different signal transduction pathways through which EPO acts. When EPO acts on EPO receptor homodimers, it induces hematopoiesis through the intracellular JAK2 signal transduction pathway. When EPO acts on heterodimers of an EPO receptor and a common β receptor (βcR), however, it induces an anti-apoptotic effect through the intracellular ERK1/2 signal transduction pathway (PNAS 101: 14907-14912, 2004).

In recent organ transplant operations, organs for transplant extracted from donors for the organ transplant operations are stored for several minutes to several tens of hours before transplantation with blood flow stopped and no oxygen supply via blood flow (ischemia). Thus, transplanted organs may be morphologically or functionally damaged when blood flow in the transplanted organs is recovered by transplantation (during reperfusion), if storage conditions such as storage temperature or preservative solution are not appropriately chosen or if a long period is required before transplantation.

Known protective agents for transplanted organs include aminobenzenesulfonic acid derivatives (WO2004/022545), 4-trifluoromethyl-pyrimidine derivatives (JPA 2005-350363), purine derivatives (JPA 2005-350364), etc.

However, the protective effect of EPO on transplanted organs has not been known. Nor protective agent for transplanted organs having a survival-promoting effect has been known.
Non-patent document 1: PNAS 100: 4802-4806, 2003.
Non-patent document 2: PNAS 101: 14907-14912, 2004.
Patent document 1: WO2004/022545.
Patent document 2: JPA 2005-350363.
Patent document 3: JPA 2005-350364.

### DISCLOSURE OF THE INVENTION

### PROBLEMS TO BE SOLVED BY THE INVENTION

The present invention aims to provide novel compositions for protecting transplanted organs, especially compositions having a survival-promoting effect on transplanted organs.

### MEANS FOR SOLVING THE PROBLEMS

As a result of close studies to attain the above object, we accomplished the present invention on the basis of the finding that erythropoietin has a protective effect on transplanted organs, especially a survival-promoting effect on transplanted organs.

Accordingly, the present invention provides the following.
(1) A composition for protecting transplanted organs, comprising erythropoietin (EPO) as an active ingredient.
(2) A composition for promoting survival of transplanted organs, comprising EPO as an active ingredient.
(3) A composition for preserving organs for transplant, comprising EPO as an active ingredient.
(4) The composition as defined in any one of (1) to (3) wherein the EPO is desialylated erythropoietin.
(5) A method for protecting transplanted organs against reperfusion damage during transplantation, which comprises the step of directly administering a composition comprising erythropoietin as an active ingredient to the transplanted organs.
(6) A method for promoting survival of transplanted organs in a recipient, which comprises the step of directly administering a composition comprising erythropoietin as an active ingredient to the transplanted organs.
(7) Use of erythropoietin for the manufacture of a medical composition for protecting transplanted organs against reperfusion damage during transplantation.
(8) Use of erythropoietin for the manufacture of a medical composition for promoting survival of transplanted organs in a recipient.

### ADVANTAGES OF THE INVENTION

As shown in the example below, the compositions of the present invention exhibit a protective effect on transplanted organs against reperfusion damage during transplantation, and a survival-promoting effect on transplanted organs in a recipient. These effects of the compositions of the present invention can be obtained by directly administering EPO to organs to be transplanted. Especially, excellent effects could be observed with asialoEPO.

They may also be expected to protect transplanted organs and promote their survival after transplantation by using them as compositions for preserving organs for transplant.

### BRIEF DESCRIPTION OF THE DRAWING

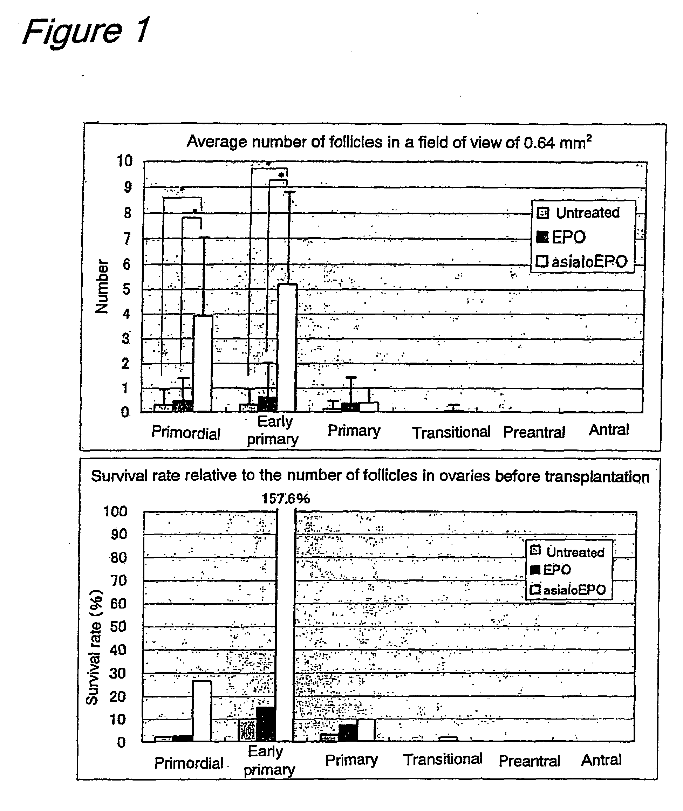

Figure 1 represents graphs showing the average number of follicles in a field of view of 0.64 mm² in transplanted ovaries and the survival rate relative to the number of follicles in ovaries before transplantation. Results are shown as mean values ± standard deviation. Asterisks (*) mean significant difference between the test groups (*: p < 0.05).

### THE MOST PREFERRED EMBODIMENTS OF THE INVENTION

Erythropoietin The erythropoietin used in the present invention can be any erythropoietin, but preferably highly purified erythropoietin, more specifically mammalian EPO, especially having biological activity substantially identical to that of human EPO.

The erythropoietin used in the present invention can be those prepared by any process, including e.g., natural human EPO purified from human-derived extracts (JPB HEI 1-38800, etc.), or human EPO recombinantly produced in E.coli, yeast cells, Chinese hamster ovary cells (CHO cells), C127 cells, COS cells, myeloma cells, BHK cells, insect cells or the like and extracted and isolated/purified by various methods. The erythropoietin used in the present invention is preferably recombinantly prepared, preferably by using mammalian cells (especially CHO cells) (e.g., JPB HEI 1-44317, Kenneth Jacobs et al., Nature, 313 806-810 (1985), etc.).

Recombinantly obtained erythropoietin may have an amino acid sequence identical to that of naturally derived EPO or may contain a deletion, substitution, addition or other modification of one or more amino acids in the amino acid sequence so far as it has similar biological activity to that of naturally derived EPO. Amino acid deletion, substitution, addition or other modification can be performed by methods known to those skilled in the art. For example, a polypeptide functionally comparable to EPO can be prepared by those skilled in the art by introducing an amino acid variation into EPO as appropriate via site-directed mutagenesis (Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz, H.J. (1987) Methods Enzymol. 154, 350-367; Kunkel, T.A. (1985) Proc. Natl. Acad. Sci. USA. 82, 488-492; Kunkel (1988) Methods Enzymol. 85, 2763-2766) or other techniques. Amino acid variations may also occur in nature. Generally, an amino acid residue is preferably substituted by another amino acid residue in which the property of the amino acid side chain is conserved. For example, the properties of amino acid side chains include hydrophobic amino acids (A, I, L, M, F, P, W, Y, V), hydrophilic amino acids (R, D, N, C, E, Q, G, H, K, S, T), amino acids having aliphatic side chains (G, A, V, L, I, P), amino acids having hydroxyl-containing side chains (S, T, Y), amino acids having sulfur-containing side chains (C, M), amino acids having carboxylate- and amide-containing side chains (D, N, E, Q), amino acids having base-containing side chains (R, K, H), and amino acids having aromatic-containing side chains (H, F, Y, W) (examples shown by one-letter amino acid codes within parentheses). It has already been known that polypeptides having an amino acid sequence modified by deleting, adding and/or substituting one or more amino acid residues retain their biological activity (Mark, D.F. et al., Proc. Natl. Acad. Sci. USA (1984) 81, 5662-5666; Zoller, M.J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413).

Fusion proteins of EPO and another protein can also be used as the erythropoietin used in the present invention. Fusion proteins can be prepared by e.g. ligating the DNA encoding EPO in-frame with the DNA encoding another protein, inserting the ligation product into an expression vector and expressing it in a host. The another to be fused to erythropoietin in the present invention is not specifically limited.

Chemically modified EPO can also be used as the erythropoietin used in the present invention. Examples of chemically modified EPO include, for example, EPO chemically modified with polyethylene glycol, etc. (WO90/12874 etc.); non-glycosylated EPO chemically modified with polyethylene glycol, etc.; and EPO conjugated to a compound such as an inorganic or organic compound, e.g., vitamin B12, etc.

EPO derivatives can also be used as the erythropoietin of the present invention. As used herein, EPO derivative refers to EPO containing a modified amino acid in the EPO molecule or to EPO containing a modified carbohydrate chain in the EPO molecule.

Modifications of carbohydrate chains in EPO molecules include addition, substitution, deletion and the like of carbohydrate chains. Preferred carbohydrate modifications in the present invention include deletion of sialic acids in EPO molecules.

Normally, both of EPO produced by recombinant animal cells and EPO derived from urine are obtained as EPO compositions containing various EPO molecules having different carbohydrate structures. The number of sialic acids attached to the EPO molecules in the EPO compositions depends on the specific EPO molecules, but normally 11 to 15 sialic acids are attached to one EPO molecule. Desialylated EPO (asialoEPO) can be prepared by removing these sialic acids. The number of sialic acids removed by desialylation is not specifically limited, and all sialic acids may be removed, or 1, 2, 3, 4, 5, 6, 7, 8, 9, 10, 11, 12, 13 or 14 sialic acids may be removed. Preferred asialoEPO in the present invention has 10 or less, more preferably 5 or less, most preferably 2 or less sialic acids attached to the EPO molecule. It should be noted that the number of sialic acids used in the present invention is the average number in EPO molecules contained in EPO compositions. The average number of sialic acids per molecule can be determined by methods known to those skilled in the art (EP 0428267, etc.).

Desialylated EPO (asialoEPO) can be prepared by methods known to those skilled in the art, e.g., by treating EPO with an enzyme such as sialidase. Sialidases are commercially available (JPA 2005-507426, Nobuo Imai et al., Eur. J. Biochem, 194, 457-462 (1990), etc.).
Glycosylated EPO can also be used as the erythropoietin used in the present invention, including EPO analogs such as NESP (Novel Erythropoietin Stimulating Protein: described in WO85/02610, WO91/05867, WO95/05465, etc.), i.e., glycosylated EPO having N-terminal sialic acid residues.

Modifications of amino acids in EPO molecules include carbamylation, biotinylation, amidination, acetylation, guanidinylation, etc.

Amino acid residues modified are not limited and include e.g., lysine, arginine, glutamic acid, tryptophan, etc. Compositions The compositions of the present invention can contain suspending agents, solubilizers, stabilizers, isotonizing agents, preservatives, adsorption inhibitors, surfactants, diluents, excipients, pH modifiers, soothing agents, buffers, sulfur-containing reducing agents, antioxidants, etc., as appropriate.

Examples of suspending agents include methylcellulose, Polysorbate 80, hydroxyethylcellulose, gum acacia, gum tragacanth powder, sodium carboxymethylcellulose, polyoxyethylene sorbitan monolaurate, etc.

Solubilizers include polyoxyethylene hardened castor oil, Polysorbate 80, nicotinic acid amide, polyoxyethylene sorbitan monolaurate, Macrogols, castor oil fatty acid ethyl esters, etc.

Stabilizers include dextran 40, methylcellulose, gelatin, sodium sulfite, sodium metasulfite, etc.

Certain amino acids can also be included as stabilizers (e.g., JPA HEI 10-182481, etc.). Amino acids added as stabilizers include free amino acids and salts thereof such as sodium salts, potassium salts, hydrochlorides, etc. Amino acids can be added alone or as a combination of two or more. Amino acids added as stabilizers are not specifically limited, but preferred amino acids include leucine, tryptophan, serine, glutamic acid, arginine, histidine and lysine.

Isotonizing agents include e.g., D-mannitol, sorbitol, etc.

Preservatives include e.g., methyl paraoxybenzoate, ethyl paraoxybenzoate, sorbic acid, phenol, cresol, chlorocresol, etc.

Adsorption inhibitors include e.g., human serum albumin, lecithin, dextran, ethylene oxide/propylene oxide copolymers, hydroxypropylcellulose, methylcellulose, polyoxyethylene hardened castor oil, polyethylene glycol, etc.

Typical examples of surfactants include: nonionic surfactants, e.g., sorbitan fatty acid esters such as sorbitan monocaprylate, sorbitan monolaurate, sorbitan monopalmitate; glycerin fatty acid esters such as glycerin monocaprylate, glycerin monomyristate, glycerin monostearate; polyglycerin fatty acid esters such as decaglyceryl monostearate, decaglyceryl distearate, decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, polyoxyethylene polyoxypropylene cetyl ether; polyoxyethylene alkyl phenyl ethers such as polyoxyethylene nonyl phenyl ether; polyoxyethylene hardened castor oils such as polyoxyethylene castor oil, polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; polyoxyethylene fatty acid amides such as polyoxyethylene stearic acid amide, each of which has an HLB of 6-18; anionic surfactants, e.g., alkyl sulfates having a C10-18 alkyl group such as sodium cetyl sulfate, sodium lauryl sulfate, sodium oleyl sulfate; polyoxyethylene alkyl ether sulfates having an average ethylene oxide mole number of 2-4 and a C10-18 alkyl group such as sodium polyoxyethylene lauryl sulfate; alkyl sulfosuccinic acid ester salts having a C8-18 alkyl group such as sodium laurylsulfosuccinate; and natural surfactants, e.g., lecithin, glycerophospholipids; sphingophospholipids such as sphingomyelin; sucrose fatty acid esters of C12-18 fatty acids. Formulations of the present invention can contain one or more of these surfactants in combination. Preferred surfactants are polyoxyethylene sorbitan fatty acid esters such as Polysorbate 20, 40, 60 or 80, especially Polysorbates 20 and 80. Polyoxyethylene polyoxypropylene glycols such as poloxamers (e.g. Pluronic F-68®) are also preferred.

Sulfur-containing reducing agents include e.g., sulfhydryl-containing compounds such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having 1 to 7 carbon atoms.

Antioxidants include e.g., erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate.

Other components commonly added may also be contained, e.g., inorganic salts such as sodium chloride, potassium chloride, calcium chloride, sodium phosphate, potassium phosphate and sodium bicarbonate; and organic salts such as sodium citrate, potassium citrate and sodium acetate.

The content of each active ingredient in the erythropoietin-containing compositions of the present invention is appropriately determined in consideration of the condition and size of the organ to be transplanted, the condition, weight, age and sex of the patients (donor and recipient), etc. Normally, it is about 10 to 1000 U/kg weight, preferably 100 to 600 U/kg weight.

The erythropoietin-containing compositions of the present invention can be administered to recipients during and/or before or after transplant operations, but the effects of the present invention can preferably be obtained by direct administration to organs to be transplanted. They can be administered by directly contacting an organ extracted from a donor with a composition of interest before transplantation or using a sponge or the like soaked with it as described in the example below. They can also be used as preservative solutions before transplantation. Additives that can be contained in the preservative solutions include physiologically acceptable buffers such as physiological saline, phosphate-buffered physiological saline and citrate buffer and isotonic solutions. The compositions can also be used in combination with conventional solutions clinically used for preserving organs for transplant such as Euro-Collins solution and UW solution.

### EXAMPLE

Example 1: Effects of administration of EPO and asialoEPO during heterologous transplantation of frozen-thawed dog ovaries
(1) Purpose
   A report shows that when frozen-thawed dog ovaries were heterologously transplanted into ovarian bursae of NOD-SCID mice, the dog ovaries survived with high frequency but 70% of primordial follicles died before revascularization after transplantation, and homologous transplantation tests in dogs also show that primordial follicles and other follicles tended to decrease in dog ovaries as compared with before transplantation.
   Thus, EPO and asialoEPO were topically administered to evaluate their inhibitory effects against the decrease of primordial follicles in dog ovaries after transplantation.
(2) Materials and methods
   The ovaries from a 4-month-old dog were frozen-thawed and transplanted into ovarian bursae of NOD-SCID mice. The freezing/thawing procedures were performed according to the method of Migishima et al. (Migishima F et al., Biol Reprod 2003 Mar; 68(3):881-7), and ovarian transplantation was performed according to the method of Ishijima et al. (Ishijima T et al., J Reprod Dev. 2006 Apr; 52(2):293-9).
(2.1) Freezing procedure
   Dog ovarian tissue was minced into 1.0 mm - 1.5 mm cubes, which were immersed in 1 M DMSO at room temperature for about 60 seconds and then placed in a cryotube containing 5 µl of 1 M DMSO, and the tube was cooled on ice for 5 minutes. After 95 µl of DAP 213 precooled on ice was added, the tube was cooled on ice for 5 minutes and immersed in liquid nitrogen.
(2.2) Thawing procedure
   The tube was removed from liquid nitrogen, and liquid nitrogen in the tube was discarded and the tube was allowed to stand at room temperature for 60 seconds. To the tube was added 900 µl of 0.25 M sucrose prewarmed at 37°C and the suspension was quickly stirred by mild pipetting and washed with PBI five times.
   A part of the extracted dog ovaries were fixed in 10% formalin to prepare pre-transplant ovarian tissue samples.
(2.3) Transplantation method
   Mice (n=9) were anesthetized by intraperitoneal administration of Nembutal and then the dorsal skin was incised to draw out the ovaries. An incision was made in the fat on the lateral side of each ovary to remove the mouse ovary in the ovarian bursa, leaving a part to ensure blood flow to a dog ovarian xenograft after transplantation, and the frozen-thawed dog ovarian tissue was placed there (in the ovarian bursa). A hemostatic sponge (hereinafter referred to as spongel) soaked with 400 U/kg of EPO (n=3) or asialoEPO (n=3) was also placed in the ovarian bursa. As a control, a spongel soaked with an equivalent amount of physiological saline was placed in the ovarian bursa.
   After 4 weeks, the transplanted ovaries were removed and fixed in 10% formalin and subjected to HE staining together with the pre-transplant ovarian tissue samples.
(2.4) Preparation of sections and follicle analysis
   Follicles that visibly contained an ovum (oocyte) with a nucleus were counted according to the classification of Oktay et al. (Oktay K et al., Biol Reprod. 1995 Aug; 53(2): 295-301) as follows.
   Primordial represents follicles containing an oocyte partially or completely encapsulated by squamous pregranulosa cells; Early primary represents follicles in which at least one of the pregranulosa cells became columnar (enlarged); Primary represents follicles in which all granulosa cells show enlargement and having a single layer of granulosa cells; Transitional represents follicles containing an oocyte encapsulated by 1-2 layer of columnar granulosa cells; Preantral represents follicles containing an oocyte encapsulated by more than 2 layers of granulosa cells with no antrum formation; Antral represents follicles containing an oocyte encapsulated by more than 2 layers of granulosa cells with antrum formation.
(2.4.1) Pre-transplant ovarian tissue
   Ten tissue samples were randomly selected and the number of follicles in the ten tissue samples was counted. The number of follicles in a circle of 900 µm in diameter, i.e., a field of view of 0.64 mm², containing the highest number of follicles in each of the selected tissue samples was counted (in a total of 10 fields of view). This number was recorded as the number of follicles before transplantation.
(2.4.2) Transplanted ovarian tissue
   Five sections were sequentially prepared for a tissue specimen (a block). The distance between sections was 40 µm to 50 µm. The number of follicles in a circle of 900 µm in diameter, i.e., a field of view of 0.64 mm², containing the highest number of follicles in each section was counted (in a total of 5 fields of view).
(3) Results

The results are shown in Table 1 and Figure 1.

Table 1

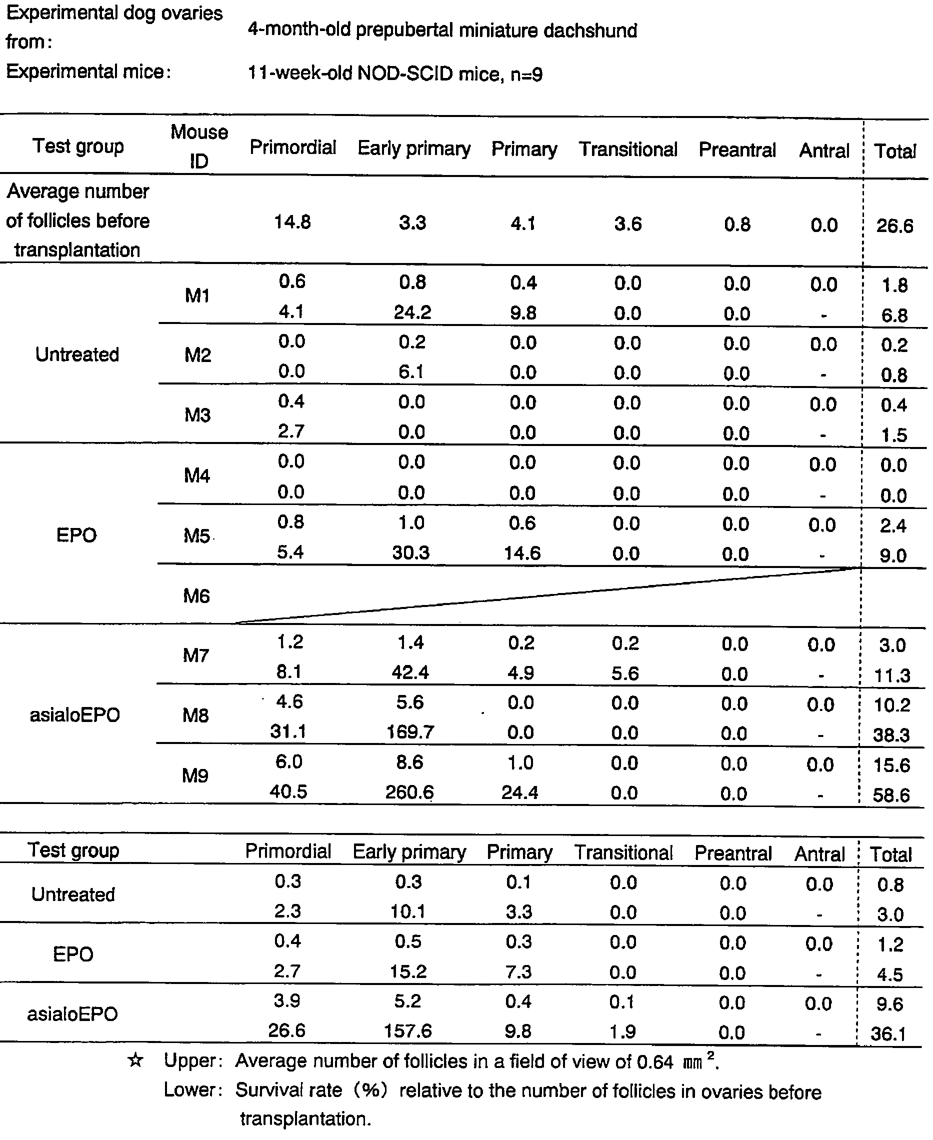

The average number of primordial follicles per 0.64 mm² in ovaries was 0.3 in the untreated group, 0.4 in the EPO group, and 3.9 in the asialoEPO group at week 4 post-transplantation in contrast to 14.8 before transplantation, which means that especially the asialoEPO group showed a significantly higher survival rate (27%) as compared with the untreated group (2.3%).

The survival rate of early primary follicles was 15.2% in the EPO group and 157.6% in the asialoEPO group in contrast to 10.1% in the untreated group. This clearly suggests that primordial follicles have partially grown into early primary follicles especially in the asialoEPO group. Moreover, it was found that especially the asialoEPO group had a tendency to show higher survival rates of primary follicles and transitional follicles as compared with the untreated group.

These results demonstrated that administration of EPO, especially asialoEPO could be effectively used for survival of the tissues of transplanted organs.

## Claims

1. A composition for protecting transplanted organs, comprising erythropoietin as an active ingredient.

2. A composition for promoting survival of transplanted organs, comprising erythropoietin as an active ingredient.

3. A composition for preserving organs for transplant, comprising erythropoietin as an active ingredient.

4. The composition of any one of claims 1 to 3 wherein the erythropoietin is desialylated erythropoietin.

5. A method for protecting transplanted organs against reperfusion damage during transplantation, which comprises the step of directly administering a composition comprising erythropoietin as an active ingredient to the transplanted organs.

6. A method for promoting survival of transplanted organs in a recipient, which comprises the step of directly administering a composition comprising erythropoietin as an active ingredient to the transplanted organs.

7. Use of erythropoietin for the manufacture of a medical composition for protecting transplanted organs against reperfusion damage during transplantation.

8. Use of erythropoietin for the manufacture of a medical composition for promoting survival of transplanted organs in a recipient.
